# EUROPÄISCHE PATENTSCHRIFT

(11) **EP 0 425 888 B1**
(45) Veröffentlichungstag und Bekanntmachung des Hinweises auf die Patenterteilung: **18.11.1993**
(21) Anmeldenummer: 90119821.8
(22) Anmeldetag: 16.10.1990
(51) Int. Cl.: C07C 23/08, C07C 17/00

(54) **Verfahren zur Herstellung von Polychlor-fluor-cyclopentenen**
Process for the preparation of polychloro-fluor-cyclopentenes
Procédé pour la préparation de polychloro-fluor-cyclopentènes

(30) Priorität: 28.10.1989 DE 3936023
(43) Veröffentlichungstag der Anmeldung: 08.05.1991
(73) Patentinhaber: BAYER AG, 51368 Leverkusen (DE)
(72) Erfinder: Schwarz, Hans-Helmut, Dr., W-4150 Krefeld (DE); Braden, Rudolf, Dr., D-5068 Odenthal (DE); Marhold, Albrecht, Dr., W-5090 Leverkusen 1 (DE); Negele, Michael, Dr., W-5000 Köln 80 (DE)

(56) Entgegenhaltungen:
- DE-A- 1 900 241
- US-A- 2 449 233

## Beschreibung

Die vorliegende Erfindung betrifft ein Verfahren zur Herstellung von Polychlor-fluor-cyclopentenen durch Umsetzung halogenhaltiger cyclischer 5-Ring-Verbindungen mit Fluorwasserstoff in der Gasphase an einem Katalysator, das dadurch gekennzeichnet ist, daß man Hexachlorcyclopentadien als Ausgangsmaterial, 7 bis 40 Mol Fluorwasserstoff, bezogen auf ein Mol Hexachlorcyclopentadien, und einen Katalysator einsetzt dessen wesentlicher Wirkstoff eine Verbindung von Wismut, von einem Lanthanid oder von Chrom ist und bei 300 bis 450°C in Gegenwart von 0,1 bis 2 Mol Chlor, bezogen auf ein Mol Hexachlorcyclopentadien arbeitet.

Die bekannten Verfahren, Polychlor-fluor-cyclopentene (PCFCP) durch Umsetzung halogenhaltiger cyclischer 5-Ring-Verbindungen mit HF in der Gasphase an Katalysatoren herzustellen, benutzen als Ausgangsmaterial entweder Octachlorcyclopenten (DE-OS 15 43 015) oder teilfluorierte, an der Doppelbindung chlorierte Chlor-fluor-cyclopent-1-ene mit mindestens einem Fluoratom im Molekül (US 3 178 482 ; US 3 258 500). Als Katalysatoren für solche Umsetzungen werden Aktivkohlen (US 3 178 482), aktivierte Chromgele (US 3 258 500) oder mit Kupfer oder Kobalt dotiertes Aluminiumoxid (DE-OS 15 43 015) eingesetzt. Die genannten Verfahren erlauben nur mäßige Umsätze zu PCFCP und gehen von Ausgangsprodukten aus, die selbst nur über mehrstufige Synthesen zugänglich sind.

Hexachlorcyclopentadien wird gemäß US 2 449 233 mit SbF₅ bzw. mit in situ aus SbCl₅ und HF/Cl₂ erzeugtem SbF₅ in der Flüssigphase unter Druck in PCFCP übergeführt. Dieses Verfahren ist aufwendig und erfordert große Mengen teurer und umweltbelastender Sb-Verbindungen.

In DE-OS 19 00 241 wird die Hydrofluorierung von teilweise und vollständig chlorierten Olefinen in der Gasphase an Trägerkatalysatoren auf Al₂O₃ beschrieben, die zu den entsprechenden gesättigten Fluoralkanen führen soll. In dieser DE-OS wird auf Seite 6. Zeile 8, als cyclischer Halogenkohlenwasserstoff Hexachlorcyclopentadien genannt, es fehlt jedoch eine Beschreibung der Umsetzungsbedingungen und der entstehenden Produkte; so wird vor allem kein PCFCP als Produkt genannt.

Aus DE-OS 20 57 398 ist weiterhin bekannt, daß aliphatische Chlorkohlenwasserstoffe bei Abwesenheit von Katalysatoren mit Cl₂ und HF bei Temperaturen von 400 bis 800°C zu Tetrachlormethan bzw. Fluorchlormethanen zu spalten sind. Dies gilt gemäß Seite 3. Zeile 22, dieser DE-OS auch für perchlorierte Cycloaliphaten.

Somit war es überraschend, daß das vorliegende erfindungsgemäße Verfahren den Chlor-Fluor-Austausch an Hexachlorcyclopentadien unter Bildung verschieden hoch fluorierter halogenierter Cyclopentene erlaubt. In DE-OS 1 543 015 ist nämlich auf Seite 12, Zeile 13, beschrieben, das sich höher (vollständig) halogenierte (Cyclo)-Aliphaten rascher fluorieren lassen, sodaß diese DE-OS das umständlicher herzustellende Octachlor-cyclopenten für notwendig hält, welches jedoch auch nur mäßige Ergebnisse liefert. Das Ausgangsprodukt Hexachlorcyclopentadien ist eine bequem herstellbare, technisch zugängliche Verbindung (Ullmanns Enzyklopädie der technischen Chemie, 5. Aufl., Bd. A8 (1987), 231; Chem. Rev. 58 (1958), 250).

Zur Umsetzung wird Hexachlor-cyclopentadien im dampfförmigen Zustand und im Gemisch mit HF und Cl₂ in ein Katalystorbett eingeleitet. Die Reaktion kann im Festbett oder in der Wirbelschicht durchgeführt werden. Die Reaktionstemperatur liegt im Bereich von 300-450°C, bevorzugt von 320-420°C. In der Regel wird unter Normaldruck gearbeitet.

Zur Umsetzung werden 7-40 Mol, bevorzugt 10-20 Mol HF pro Mol des Ausgangsmaterials eingesetzt. Überschüssiges HF, das den Reaktor unverbraucht verläßt, wird vorteilhafterweise recyclisiert.

Es wird in Gegenwart von 0,1 bis 2 Mol Chlor, bevorzugt 0,3 bis 1,1 Mol Chlor, pro Mol des Ausgangsmaterials gearbeitet.

Erfindungsgemäß wird an einem Katalysator gearbeitet, dessen wesentlicher Wirkstoff eine Verbindung von Wismut, einem Lanthanid oder Chrom ist. Besonders bevorzugt sind Katalysatoren mit einem Gehalt an Wismut oder einem Lanthanid.

Zu solchen besonders bevorzugten Wismutkatalysatoren gehören solche mit Verbindungen der Erdalkalimetalle, die 0,005 - 0,8 gA Wismut, bevorzugt 0,008-0,7 gA Wismut. pro Mol der Trägerverbindung aufweisen und weiterhin Promotoren undloder inerte Zusatzstoffe (beispielsweise Graphit) enthalten konnen. Eine wesentliche Komponente dieser Katalystoren sind Erdalkaliverbindungen, die aus mindestens einer Verbindung aus der Gruppe der Carbonate, Hydroxide, Oxide und Fluoride der Erdalkalimetalle, in bevorzugter Weise solche, die aus mindestens einer Verbindung aus der Gruppe Magnesiumoxid, Calciumoxid und Bariumoxid hervorgegangen sind. Solche Wismutkatalysatoren konnen weiterhin als Promotoren mindestens eine Verbindung von Elementen der VII. und VIII. Nebengruppe des Periodensystems der Elemente (Mendelejew) oder der Lanthanide enthalten: das Grammatomverhältnis der Promotorelemente zum Element Wismut beträgt hierbei 0,01-200:1, bevorzugt 0,1-10:1. Den Katalysatoren konnen inerte Zusatzstoffe, beispielsweise Graphit, zugesetzt werden. In bevorzugter Weise sind solche Katalysatoren frei von inerten Zusatzstoffen.

Diese Katalysatoren können nach bekannten Verfahren hergestellt werden, beispielsweise durch bloßes Anteigen der Wirkstoffe mit möglichst wenig Wasser und inniges Vermischen der so erhaltenen pastösen Masse. Aus einer solchen Paste kann beispielsweise ein Granulat hergestellt und getrocknet werden. Im getrockneten Zustand wird ein so hergestellter Katalysator vorteilhafterweise bei einer Temperatur von 20 - 500°C, bevorzugt 100 - 500°C, besonders bevorzugt 120 - 420°C, mit überschüssigem Fluorwasserstoff behandelt. Zwischen dem Trocknungsvorgang und dem Behandlungsvorgang mit Fluorwasserstoff kann eine Temperung bei 200 - 400°C, bevorzugt 300 - 375°C eingeschoben werden.

Zu den besonders bevorzugten Katalysatoren auf der Basis von Lanthaniden als Wirkstoffen zählen solche aus mindestens einer oxidischen Verbindung von Lanthaniden oder von Verbindungen der Lanthaniden auf Katalysatorträgern. Bevorzugt Lanthanide sind Cer, Lanthan und Dysprosium. Der Katalysatorträger kann beispielsweise Al₂O₃, AlF₃, Aktivkohle, Koks, CaF₂ oder ZnF₂ sein. Auch solche Katalysatoren werden nach bekannten Methoden, beispielsweise durch das oben beschriebene Anteigen der Einsatzstoffe hergestellt und in bevorzugter Weise nach der Trocknung bei einer Temperatur von 20 - 500°C, bevorzugt 100 - 500°C, besonders bevorzugt 120 - 420°C mit überschüssigem Fluorwasserstoff behandelt. Auch bei dieser Gruppe von Katalysatoren kann zwischen die Vorgänge der Trocknung und der Behandlung mit Fluorwasserstoff eine Temperung bei 200 - 400°C, bevorzugt 300 - 375°C eingeschoben werden. Die Verbindungen der Lanthaniden betragen 0,1 - 100 Gew.-% des Gesamtgewichts des Katalysators, wobei Gewichtsanteile unter 100 % für den Fall der Mitverwendung von Katalysatorträgern gelten. Für diesen Fall der Mitverwendung von Katalysatorträgern beträgt das Gewicht der Verbindungen der Lanthaniden in bevorzugter Weise 2 - 30 Gew.-% des Gesamtgewichts des Katalysators.

Polychlor-fluor-cyclopentene besitzen technische Bedeutung als Zwischenprodukte für Kunststoffvorprodukte.

### Beispiel 1

2,5 Gew.-Teile Bi(NO₃)₃.5 H₂O und 1,2 Teile 65 %ige Salpetersäure wurden in 3,6 Teilen Wasser gelöst. Dazu gab man 1 Teil Fe(NO₃)₃.9 H₂O. Diese Lösung wurde zu 1,5 Gew.-Teilen Magnesiumoxid gegeben und die dabei entstandene pastöse Masse innig verknetet. Anschließend wurde das pastöse Reaktionsprodukt granuliert und 16 h bei 100°C getrocknet. Danach wurde der Katalysator 6 h auf 400°C erhitzt. Das Atomverhältnis Mg:Bi:Fe betrug 1:1,29:0,17.

0,33 l der Kontaktkörper wurden in einem Rohr mit 5 cm lichter Weite und 100 cm Länge bei 350°C mit 5 Mol Fluorwasserstoff (HF) behandelt. Die Dauer der HF-Behandlung betrug ca. 3 h. Dabei wurde HF mit N₂ im Molverhältnis 1:2 verdünnt.

Über 200 ml dieses Katalysators wurden im Laufe von 5 h 102 g Hexachlorcyclopentadien, 210 g HF und 9,5 g Chlor bei 350°C geleitet. Die Reaktionsgase wurden in einem Eis-Wasser-Gemisch kondensiert. Es schieden sich 72,4 g einer organischen Phase ab, die nach gaschromatographischer (GC) Analyse 58,4 % 1,2-Dichlor-3,3,4,4,5,5-hexafluorcyclopenten-1, 29,1 % trichlorierte Cyclopentene (1,2,4-Trichlor-3,3,4,5,5-pentafluorcyclopenten-1 und Isomere mit 1,2-Chlorierung) und 8,1 % tetrachlorierte Cyclopentene (1,2,4,4-Tetrachlor-3,3,5,5-tetrafluorcyclopenten-1 und Isomere mit 1,2-Chlorierung) enthielten. Hexachlorcyclopentadien hatte sich vollkommen umgesetzt.

### Beispiel 2

Entsprechend dem Beispiel 1 wurde ein Katalysator aus 162,8 g Ce(NO₃)₃.6 H₂O, 255 g Wasser und 500 ml Aluminiumoxid SAS 350 (Fa. Rhone-Poulenc) hergestellt und aktiviert.

Über 120 ml dieses Katalysators wurden im Laufe von 5 h bei 410°C 100 g Hexachlorcyclopentadien, 200 g HF und 9 g Chlor geleitet.

Bei der Absporption der Reaktionsgase in Eiswasser entstanden 87,5 g einer organischen Phase, die 2,1 % 1,2-Dichlor-3,3,4,4,5,5-hexafluorcyclopenten-1, 23,8 % 1,2,4-Trichlor-3,3,4,5,5-pentafluorcyclopenten-1 (und Isomere wie Beispiel 1), 35,1 % 1,2,4,4-Tetrachlor-3,3,5,5-tetrafluorcyclopenten-1 (und Isomere wie Beispiel 1) sowie 3 % Hexachlorcyclopentadien enthielt.

### Beispiel 3

1000 ml Al₂O₃ des Typs SAS 350 (Fa. Rhone-Poulenc) in Form von 3-6 mm Kugeln wurden mit einer wäßrigen Lösung imprägniert, die 2,1 Mol Chrom-III-chlorid enthielt. Die Katalysatorkugeln wurden getrocknet und bei 200-250°C mit einem dampfförmigen NH₃-H₂O-Gemisch behandelt. Im Laufe von 4 h wurden hierzu 4 Mole NH₃ in Form einer 30 %igen wäßrigen Lösung verdampft und über den Katalysator geleitet.

Über 140 ml dieses Katalysators wurden bei 360°C im Laufe von 6 h 200 g HF geleitet. Anschließend wurden bei 350°C während 5 h 102 g Hexachlorcyclopentadien, 170 g HF und 23 g Chlor geleitet. Die Reaktionsgase wurden in Eiswasser kondensiert. Es bildeten sich 85,4 g einer organischen Phase, die 60,5 % 1,2-Dichlor-3,3,4,4,5,5-pentafluorcyclopenten-1, 23,5 % 1,2,4-Trichlor-3,3,4,5,5-pentafluorcyclopenten-1 (und Isomere wie Beispiel 1) und 9,7 % 1,2,4,4-Tetrachlor-3,3,5,5-tetrafluorcyclopenten-1 (und Isomere wie Beispiel 1) enthielt. Der Hexachlorcyclopentadien-Gehalt im Reaktionsprodukt betrug 0,1 %.

### Beispiel 4

In 30 g 18 %iger Salzsäure wurden 20 g BiCl₃ und in 150 g Wasser 16,76 g FeCl₃.6 H₂O gelöst. Beide Lösungen wurden vereinigt und allmählich mit 250 g Magnesiumoxid unter weiterer Wasserzugabe von 170 ml intensiv verknetet. Das Material wurde bei 100°C i.V. getrocknet, zerkleinert und die Fraktion mit der Korngröße 2-5 mm herausgesiebt. Dieser Katalysator wurde bei 350°C 5 h durch Behandlung mit HF aktiviert.

Im Laufe von 5 h wurden 102 g Hexachlorcyclopentadien und 201,5 g HF bei 415°C über 220 ml des Katalysators geleitet. Bei der Kondensation der Reaktionsgase in kaltem Wasser entstanden 68,9 g einer organischen Phase, die 8,5 % 1,2-Dichlor-3,3,4,4,5,5-hexafluorcyclopent-1-en, 16,3 % 1,2,4-Trichlor-3,3,4,5,5-pentafluorcyclopent-1-en (und Isomere wie Beispiel 1), 23,8 % Tetrachlor-tetrafluor-cyclopentene, 18,4 % Pentachlor-trifluor-cyclopentene, 13,3 % Mono- und Difluor-polychlor-cyclopentene sowie 9,7 % Hexachlorcyclopentadien enthielt.

### Beispiel 5

Über 220 ml des Katalysators nach Beispiel 4 wurden im Laufe von 4 h 81,6 g Hexachlorcyclopentadien, 108 g HF und 10,8 g Chlor bei 415°C geleitet. Bei der Kondensation der Reaktionsgase in Wasser bildeten sich 73,6 g einer organischen Phase, die 3,8 % 1,2-Dichlor-3,3,4,4,5,5-hexafluorcyclopent-1-en, 15,6 % 1,2,4-Trichlor-3,3,4,5,5-pentafluorcyclopent-1-en (und Isomere wie Beispiel 1), 33,7 % 1,2,4,4-Tetrachlor-3,3,5,5-tetrafluorcyclopent-1-en (und Isomere wie Beispiel 1), 17,0 % Pentachlor-trifluorcyclopent-1-en, 13 % Di- und Monofluor-chlorcyclopent-1-en sowie 6,6 % Hexachlorcyclopentadien enthielt.

## Patentansprüche

1. Verfahren Zur Herstellung von Polychlor-fluor-cyclopentenen durch Umsetzung von halogenhaltiger cyclischer 5-Ring-Verbindungen mit Fluorwasserstoff in der Gasphase an einem Katalysator, dadurch gekennzeichnet, daß man Hexachlorcyclopentadien als Ausgangsmaterial, 7 bis 40 Mol Fluorwasserstoff, bezogen auf ein Mol Hexachlorcyclopentadien und einen Katalysator einsetzt, dessen wesentlicher Wirkstoff eine Verbindung von Wismut, von einem Lanthanid oder von Chrom ist, und bei 300 bis 450°C in Gegenwart von 0,1 bis 2 Mol Chlor, bezogen auf ein Mol Hexachlorcyclopentadien, arbeitet.

2. Verfahren nach Anspruch 1, dadurch gekennzeichnet, daß man in Gegenwart von 0,3 - 1,1 Mol Chlor pro Mol des Hexachlorcyclopentadien arbeitet.

3. Verfahren nach Anspruch 1 und 2, dadurch gekennzeichnet, daß der Katalysator einen Gehalt an Wismut oder einem Lanthanid hat.

4. Verfahren nach Anspruch 3, dadurch gekennzeichnet, daß Wismut in Kombination mit einer Erdalkalimetallverbindung eingesetzt wird.

5. Verfahren nach Anspruch 3, dadurch gekennzeichnet, daß das Lanthanid auf einem Träger angebracht ist, der aus mindestens einer Verbindung aus der Gruppe von Al₂O₃, AlF₃, Aktivkohle, Koks, CaF₂ oder ZnF₂ besteht.

## Claims

1. Process for the preparation of polychloro-fluorocyclopentenes by reaction of halogen-containing cyclic compounds having a 5-membered ring with hydrogen fluoride in the gas phase over a catalyst, characterized in that hexachlorocyclopentadiene as the starting material, 7 to 40 mol of hydrogen fluoride, based on one mole of hexachlorocyclopentadiene and a catalyst in which the essential active compound is a compound of bismuth, of a lanthanide or of chromium are employed and the reaction is carried out at 300 to 450°C in the presence of 0.1 to 2 mol of chlorine, based on one mole of hexachlorocyclo-pentadiene.

2. Process according to Claim 1, characterized in that the reaction is carried out in the presence of 0.3 - 1.1 mol of chlorine per mole of hexachloro-cyclopentadiene.

3. Process according to Claims 1 and 2, characterized in that the catalyst contains bismuth or a lanthanide.

4. Process according to Claim 3, characterized in that bismuth is employed in combination with an alkaline earth metal compound.

5. Process according to Claim 3, characterized in that the lanthanide is applied to a support which consists of at least one compound from the group comprising Al₂O₃, AlF₃, active charcoal, coke, CaF₂ or ZnF₂.

## Revendications

1. Procédé de production de polychloro-fluorocyclopentènes par réaction de composés halogénés cycliques pentagonaux avec du fluorure d'hydrogène en phase gazeuse sur un catalyseur, caractérisé en ce qu'on utilise de l'hexachlorocyclopentadiène comme matière de départ, 7 à 40 moles % de fluorure d'hydrogène, pour une mole d'hexachlorocyclopentadiène et un catalyseur dont la substance active essentielle est un composé de bismuth, d'un lanthanide ou de chrome, et on opère à 300-450°C en présence de 0,1 à 2 moles de chlore, pour une mole d'hexachlorocyclopentadiène.

2. Procédé suivant la revendication 1, caractérisé en ce qu'on opère en présence de 0,3 à 1,1 mole de chlore par mole d'hexachlorocyclopentadiène.

3. Procédé suivant les revendications 1 et 2, caractérisé en ce que le catalyseur contient du bismuth ou un lanthanide.

4. Procédé suivant la revendication 3, caractérisé en ce qu'on utilise du bismuth en association avec un composé de métal alcalino-terreux.

5. Procédé suivant la revendication 3, caractérisé en ce que le lanthanide est déposé sur un support qui est constitué d'au moins un composé du groupe Al₂O₃, AlF₃, charbon actif, coke, CaF₂ ou ZnF₂.
